# EUROPEAN PATENT SPECIFICATION

(11) **EP 1 157 120 B1**
(45) Date of publication and mention of the grant of the patent: **07.12.2005**
(21) Application number: 00906103.7
(22) Date of filing: 23.02.2000
(51) Int. Cl.: C12N 15/85, C12N 15/62, C12N 5/10, C07K 14/435, C07K 14/535, C12N 9/18

(54) **SEQUENCES FOR IMPROVING THE EFFICIENCY OF SECRETION OF NON-SECRETED PROTEINS FROM MAMMALIAN AND INSECT CELLS**
SEQUENZEN ZUR VERBESSERUNG DER SEKRETIONSEFFIZIENZ VON NICHT-SEKRETIERTEN PROTEINEN IN SÄUGETIER- UND INSEKTENZELLEN
SEQUENCES PERMETTANT D'AMELIORER L'EFFICACITE DE LA SECRETION DE PROTEINES NON SECRETEES DE CELLULES DE MAMMIFERES ET D'INSECTES

(30) Priority: 24.02.1999 US 256694
(43) Date of publication of application: 28.11.2001
(73) Proprietor: University Technologies International Inc., Calgary, Alberta T2L 2K7 (CA)
(72) Inventor: IATROU, Kostas Institute of Biology NCSR, Attkis Athens (GR); FARRELL, Patrick J., Calgary, Alberta T3A 2L8 (CA); BEHIE, Leo, A., Calgary, Alberta (CA)
(74) Representative: Vossius & Partner
(86) International application number: PCT/CA2000/000188
(87) International publication number: WO 2000/050616

(56) References cited:
- EP-A- 0 326 419
- EP-A- 0 608 696
- EP-A- 0 646 646
- WO-A-94/00585
- WO-A-99/10489
- DIFALCO M R ET AL: "The influence of various insect cell lines, p10 and polyhedrin promoters in the production of secreted insulin-like growth factor-interleukin-3 chimeras in the baculovirus expression system" JOURNAL OF BIOTECHNOLOGY,NL,ELSEVIER SCIENCE PUBLISHERS, AMSTERDAM, vol. 56, no. 1, 23 July 1997 (1997-07-23), pages 49-56, XP004126077 ISSN: 0168-1656
- BEI R ET AL: "Baculovirus expression of a functional single-chain immunoglobulin and its IL-2 fusion protein" J IMMUNOL METHODS, vol. 186, no. 2, 26 October 1995 (1995-10-26), pages 245-255, XP000929576
- FARRELL P ET AL: "High-level expression of secreted glycoproteins in transformed lepidopteran insect cells using a novel expression vector" BIOTECHNOLOGY AND BIOENGINEERING, vol. 60, no. 6, 20 December 1998 (1998-12-20), pages 656-663, XP002143887
- J W MARTENS ET AL: "Characterization of baculovirus insecticides expressing tailored Bacillus thurigiensis CryIA(b) crystal proteins" JOURNAL OF INVERTEBRATE PATHOLOGY,US,SAN DIEGO, CA, vol. 66, no. 3, 1 November 1995 (1995-11-01), pages 249-257, XP002089682 ISSN: 0022-2011 cited in the application
- LO K-M ET AL: "High level expression and secretion of Fc-X fusion proteins in mammalian cells" PROTEIN ENGINEERING, vol. 11, no. 6, 1998, pages 495-500, XP002143888

## Description

### BACKGROUND OF THE INVENTION

### FIELD OF THE INVENTION

The present invention relates to the engineering of heterologous gene constructs by recombinant DNA techniques for the more efficient processing and secretion of heterologous genes in mammalian and insect cells. Particularly the present invention relates to the use of a secretion competent polypeptide linked in frame with a non-secretion competent polypeptide to direct the secretion of the non-secretion competent polypeptide, wherein said secretion competent polypeptide comprises the complete amino acid sequence of insect juvenile hormone esterase (JHE) or a portion thereof, wherein said portion is capable of directing secretion of a non-secretion competent heterologous protein at a higher rate than the mere signal peptide of said JHE.

### DESCRIPTION OF THE RELATED ART

Recombinant polypeptides for medical, research and veterinary applications are produced using a wide variety of genetically engineered organisms that include transgenic animals (eg. cows, goats) transgenic plants (eg. canola) recombinant viruses (eg. baculoviruses) and transformed prokaryotic cells (eg: bacteria) and eukaryotic cells (eg. yeast and animal cells) in culture.

Since most of the proteins are glycoproteins requiring advanced post-translational modification expression systems using yeast and bacteria are unsuitable. For this reason, other protein expression systems were developed using higher eukaryotes, including virus-based expression systems such as baculovirus and adenovirus and expression from transformed mammalian cells (CHO, BHK NsO etc. and production in the milk of transgenic farm animals). However, even these most advanced vehicles for protein production are inadequate due to difficulties in recovery and purification of the recombinant proteins.

Viral expression systems can produce impressive levels of recombinant proteins in both insect (Maiorella et al. 1988) and mouse cell lines (Garnier et al. , 1994) but suffer from serious biological and engineering disadvantages. First, because host cells are killed at the end of each infection cycle, protein expression is only temporary. This also means that protein expression is not suited to the state of the art perfusion bioreactors. Second, the biological authenticity of the expressed protein is not guaranteed because the cell machinery necessary for post-translational modifications is inactivated in the late stages of infection. Unsuitable N-linked glycosylation patterns are widely reported for proteins produced following infection with recombinant viruses, which alters the normal glycosylation characteristics of the cell hosts (Jarvis and Summers, 1989). It is known however that the lepidopteran insect cell hosts are capable of the complex oligosaccharide processing required for *in vivo* human use of proteins (Davis and Wood 1995) Thirdly, although native genes containing all or part of their introns are generally expressed at a higher level than the corresponding cDNAs (Brinster et al. 1988) virus infected insect cells cannot efficiently excise introns from expressed genomic DNA, thus limiting foreign protein expression from cDNAs only (O' Reilly et al., 1991). Fourth, purification of recombinant proteins from virus infected systems is problematic. Because proteins cannot be secreted efficiently in viral systems due to the inactivation of the secretory pathway upon infection (Jarvis and Summers 1989) they must be recovered from cell lysates after cell lysis. The presence of proteases in such cellular lysates also cause degradation of the over-expressed product.

A major problem in biotechnology exists in the production and recovery of recombinant non-secretion competent polypeptides, such as intracellular proteins or protein subunits, from genetically engineered organisms. Often these intracellular proteins or protein subunits can be expressed at only moderate levels inside a cell and their purification must first include steps to lyse the cells, followed by complex procedures to isolate the desired polypeptides from many other intracellular proteins.

All secreted proteins possess a consensus signal peptide of 10 to 50 amino acids at their N-terminus that directs the protein to the secretory pathway of eukaryotic cells or to the cytoplasmic membrane of prokaryotic cells. Using genetic engineering techniques, some research groups have therefore tried to secrete intracellular proteins by fusing the gene sequences of consensus signal peptides in-frame to the 5' end of the gene encoding the non-secretion polypeptide. When these heterologous genes were expressed, however, the mere presence of a consensus signal peptide was found to be insufficient for the efficient secretion of non-secretion competent polypeptides across a given biological membrane, a problem which is often encountered in the field of biotechnology. For example, Martens et al. (1995), attached the signal sequence of the juvenile hormone esterase gene to the 5' end of the CryIA(b) insecticidal crystal protein gene to induce secretion but found that secretion into the medium from the insect cells was poor.

A method to efficiently secrete non-secretion competent polypeptides, such as cytoplasmic proteins, nuclear factors and protein subunits would be desirable. This would allow the recombinant protein to be expressed at a higher level. Second because the recombinant protein would be secreted into the extracellular environment, purification of the peptide or protein would not be complicated by the presence of other intracellular proteins and would not involve harming the producing cells.

Advantages of the present invention will become apparent from the following description of the invention with reference to the attached drawings.

### SUMMARY OF THE INVENTION

The present invention is directed to an expression cassette useful for the secretion of a heterologous protein as a fusion protein comprising a polynucleotide encoding from its 5' to 3' direction: a) a promoter b) a signal peptide; c) a cell secretion competent polypeptide which comprises the complete amino acid sequence of insect juvenile hormone esterase (JHE) or a portion thereof, wherein said portion is capable of directing secretion of a non-secretion competent heterologous protein at a higher rate than the mere signal peptide of said JHE; and d) a heterologous protein wherein the polynucleotide sequence encoding for (b), (c) and (d) are linked in frame.

In a second aspect, the present invention is also directed to a vector useful for the secretion of a heterologous protein as a fusion protein comprising a polynucleotide encoding from its 5' to 3' direction: a) a promoter b) a signal peptide; c) a cell secretion competent polypeptide comprising the complete amino acid sequence of insect JHE or a portion thereof as defined above; and d) a heterologous protein wherein the polynucleotide sequences encoding (b), (c) and (d) are linked in frame.

In another aspect, the present invention is directed to an *in vitro* method of secreting a heterologous protein, comprising introducing into a cell an expression cassette comprising a polynucleotide encoding from its 5' to 3' direction: a) a promotor, b) a signal peptide; c) a cell secretion competent polypeptide which is insect JHE; and d) a heterologous protein wherein the polynucleotide sequences encoding (b), (c) and (d) are linked in frame under conditions wherein the heterologous protein is expressed and secreted from the cell.

The present invention is also directed to an *in vitro* method of secreting a heterologous protein from mammalian cells, comprising introducing into a mammalian cell an expression cassette comprising a polynucleotide encoding from its 5' to 3' direction: a) a promoter b) a signal peptide; c) a secretion competent polypeptide which is insect juvenile hormone esterase; and d) a heterologous protein wherein the polynucleotide sequences encoding (b) (c) and (d) are linked in frame under conditions wherein the heterologous protein is expressed and secreted from the mammalian cell.

### BRIEF DESCRIPTION OF THE DRAWINGS

Figure 1 is a schematic illustration of the generic design of a DNA molecule for the secretion of either an intracellular protein or protein subunit.
Figure 2A [SEQ ID NO: 12] is a schematic illustration of the design of the DNA module using the juvenile hormone esterase (JHE) cDNA as an example of a secretion competent polypeptide to secrete the bacterial cytoplasmic protein CAT.
Figure 2B is a photograph of a Western Blot which shows the secretion of the JHE-CAT fusion protein using the secretion module described in Figure 2A.
Figure 2C shows the liberation of the CAT protein from the fusion protein when incubated with enteropeptidase.
Figure 3A [SEQ ID NO:12] is a schematic illustration of the design of the DNA molecule using the juvenile hormone esterase (JHE) cDNA as an example of a secretion competent polypeptide to secrete the insect nuclear protein BmCF1.
Figure 3B is a photograph of a Western Blot which shows the secretion of the JHE-BmCF1 fusion protein using the secretion module described in Figure 3A.
Figure 4 [SEQ ID NO:13] shows the DNA sequence of the juvenile hormone esterase (JHE) gene from *Heliothis virescens,* Genbank Accession No. J04955 (Hanzlik et al., 1989). The first translation start codon, methionine, is indicated in bold.
Figure 5 [SEQ ID NO: 12] is a schematic illustration for comparative purposes of the design of the DNA molecule using the human granulocyte macrophage colony stimulating factor (GMCSF) cDNA as an example of a secretion competent polypeptide to secrete the CAT protein.
Figure 6 [SEQ ID NO: 14] shows for comparative purposes the DNA sequence of the human granulocyte macrophage colony stimulating factor cDNA. The first translation start codon, methionine, and the translation stop codon are indicated in bold.
Figure 7A is a photograph of a Western Blot which shows for comparative purposes the amount of the CAT protein and the GMCSF-CAT fusion protein within the cell. Figure 7B is a photograph of a Western Blot which shows the amount of the CAT protein or the GMCSF-CAT fusion protein in the supernatant. In both figures, lane 1 is cells transfected with pIE1/153A, lane 2 is cells transfected with pIE1/153A.CAT and lane 3 is cells transfected with pIE1/153A.GMCSF.HisEP.CAT.

### DESCRIPTION OF THE PREFERRED EMBODIMENTS

The present invention relates to an expression cassette useful for the secretion of a heterologous gene comprising a polynucleotide encoding from its 5' to 3' direction: a) a promoter b) a signal peptide; c)a cell secretion competent polypeptide which comprises the complete amino acid sequence of insect juvenile hormone esterase (JHE) or a portion thereof, wherein said portion is capable of directing secretion of a non-secretion competent heterologous protein at a higher rate than the mere signal peptide of said JHE; and d) a heterologous protein wherein the polynucleotide sequences encoding (b), (c) and (d) are linked in frame.

The present invention is also directed to an *in vitro* method of secreting a heterologou protein, comprising transforming a cell with a expression cassette comprising a polynucleotide encoding from its 5' to 3' direction: a) a promoter b) a signal peptide; c) a cell secretion competent polypeptide which is insect JHE; and d) a heterologous protein wherein the polynucleotide sequences encoding (b) (c) and (d) are linked in frame under conditions wherein the heterologous protein is expressed and secreted from the cell.

However, prior to discussing this invention in further detail, the following terms will first be defined.

### Definitions

The term "baculovirus" is used herein as an alternative to the term "nuclear polyhedrosis virus" or "NPV". It encompasses viruses classified under subgroup A of the family of Baculoviridae. Preferably, it includes the viruses specific for the following insects: *Bombyx* sp., *Autographica* sp., *Spodoprera* sp. and other lepidoptera.

The term "expression cassette" means a polynucleotide encoding from its 5' to 3' direction: a) a promoter, b) a signal peptide; c) a cell secretion competent polypeptide comprising the complete amino acid sequence of insect JHE or a portion thereof, wherein said portion is capable of directing secretion of a non-secretion competent heterologous protein at a higher rate than the mere signal peptide of said JHE and d) a heterologous protein wherein the polynucleotide sequences encoding (b), (c) and (d) are linked in frame. The expression cassette may additionally comprise a sequence encoding mRNA termination and polyadenylation signals. The expression cassette is capable of directing the expression and secretion of the heterologous protein as a secretion competent polypeptide-heterologous protein fusion protein when the expression cassette containing the heterologous protein is introduced into a cell, such as an insect cell.

The term "vector" means nucleic acid which comprises: (1) the expression cassette, and (2) DNA sequences allowing replication and selection in bacteria, for example *E. coli.* The vector may be a plasmid, another virus or simply a linear DNA fragment. It is contemplated that the vector may be a baculovirus artificial chromosome (BVAC) as set forth in U.S. Patent Application Serial No. 09/136,419, entitled BACULOVIRUS ARTIFICIAL CHROMOSOMES AND METHODS OF USE, Attorney Docket No. 028722-171, and filed concurrently herewith, which claims priority to U.S. Provisional Patent Application Serial No.60/056,807, filed August 21, 1997, both of which are incorporated by reference herein in their entirety.

The term "baculovirus chromosome" refers to the genome of the baculovirus, which genome is circular. In a preferred embodiment, the baculovirus artificial chromosome is derived from the *B. mori* nuclear polyhedrosis virus. In another embodiment, the chromosome is derived from the *A. californica* nuclear polyhedrosis virus or any other nuclear polyhedrosis virus that contains a *lef-8* gene or *lef*-8-like gene.

Detectable markers are genes which allow detection of cells that have been transfected or infected with the gene. Detectable markers include reporter genes and selection genes. Reporter gene are genes which confer a characteristic onto the cell which is detectable. Suitable reporter genes include the gene encoding for green fluorescent protein, the β-galactosidase gene and the chloramphenicol acetyl transferase gene. Selection genes are wild-type alleles of genes that encode for enzymes which allow the cell to grow on certain media, such as media containing antibiotics. These genes include, for example, the prokaryotic hygromycin resistance and neomycin resistance genes.

The secretion competent polypeptide or SC polypeptide, or SCP, is a polypeptide which is any complete insect jwenile hormone esterase protein or any part of said protein that is not merely a consensus signal peptide that enables complete passage of the polypeptide through the secretory pathway of the cell and through the cytoplasmic membrane, wherein the secretion directed by said part is at a higher rate than that directed by the mere signal peptide of said protein.

The term "SCP gene" means that portion of a gene which encodes for a secretion competent polypeptide. The gene does not include its stop codon.

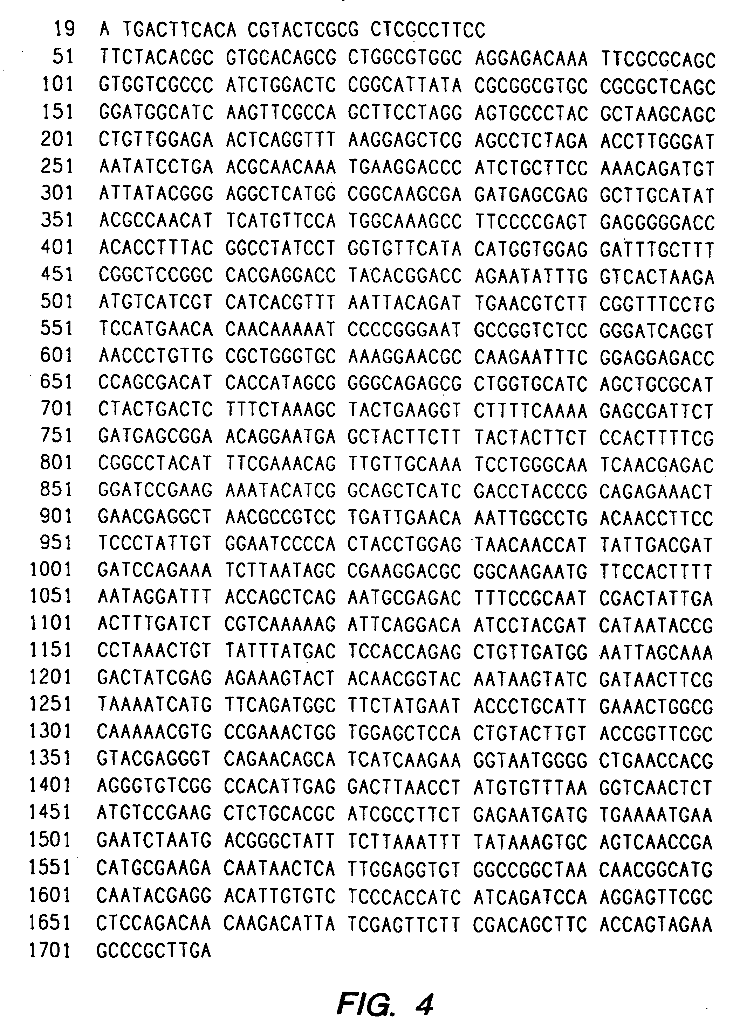

The term "juvenile hormone esterase gene" or "JHE gene" means that portion of the juvenile hormone esterase gene, in addition to its signal sequence, which encodes for a polypeptide that directs the secretion of non-secretion competent heterologous protein when the JHE gene is functionally linked in frame at its 3' end with the heterologous gene at a higher rate than the mere signal peptide of said JHE gene. The juvenile hormone esterase gene does not include its stop codon. The term "JHE gene" means a DNA sequence of at least about 100 bp, more preferably at least about 500 bp and most preferably an entire gene substantially identical to the DNA sequence as set forth in Figure 4. The juvenile hormone esterase gene is derived from *Heliothis virescens.* The JHE peptide is that portion of the JHE protein which directs the secretion of non-secretion competent heterologous protein when the JHE peptide is linked to the N-terminus of the heterologous protein at a higher rate than the mere signal peptide of said JHE protein.

The terms "producing heterologous protein" or "expressing heterologous protein" means that the structural gene encoding the heterologous protein is transcribed into mRNA and that the mRNA is further translated into protein. In a preferred embodiment the heterologous protein will be properly processed by the eukaryotic cell, although such processing may be in a tissue specific manner.

The term "secreting" or "secretion" is the active export of a protein from a cell into the extracellular environment. Generally secretion occurs through a secretory pathway in the cell, for example, in eukaryotic cells, this involves the endoplasmic reticulum and golgi apparatus.

The term "structural gene" refers to those DNA sequences which, when functionally attached to a cellular or viral promoter and linked in frame with the secretion competent polypeptide (SCP) gene, will be transcribed and produce a SCP-heterologous fusion protein which is secreted from the cells.

The term "heterologous structural gene" or "heterologous gene" is a structural gene which will be transcribed and will produce a heterologous protein when functionally attached to any promoter capable of functioning in the host cell or to an enhancer and promoter where the structural gene is introduced into eukaryotic cells either by infection or transfection of cells.

The term "heterologous protein" refers to a protein encoded by a heterologous structural gene. Examples of heterologous proteins are chloramphenicol acetyl transferase, human alpha interferon (IFN-α), insulin-like growth factor-II (IGF-II), human interleukin 3, mouse interleukin 3, human and mouse interleukin 4, human T-lymphotropic virus (HTLV-1) p40^{x}, HTLV-1 *env,* human immunodeficiency virus (HIV-1) *gag, pol, sor,* gp41, and gp120, adenovirus E1a, Japanese encephalitis virus *env* (N), bovine papilloma virus 1 (BPV1) E2, HPV6b E2, BPV1 E6, and human apolipoproteins A and E; β-galactosidase, hepatitis B surface antigen, HIV-1 *env,* HIV-1 *gag,* HTLV-1 p40^{x}, human IFN-β, human interleukin 2, *c-myc, D. melanogaster* Kruppel gene product, bluetongue virus VP2 and VP3, human parainfluenza virus hemagglutinin (HA), influenza polymerases PA, PB1, and PB2, influenza virus HA, lymphocytic choriomeningitis virus (LCMV) GPC and N proteins, *Neurospora crassa* activator protein, polyomavirus T antigen, simian virus 40 (SV40) small t antigen, SV40 large T antigen, Punta Toro phlebovirus N and Ns proteins, simian rotavirus VP6, CD4 (T4), human erythropoietin, Hantaan virus structural protein, human epidermal growth factor (EGF) receptor, human insulin receptor, human B lymphotrophic virus 130-kd protein, hepatitis A virus VP1, human tyrosine hydroxylase, human glucocerebrosidase, p53 protein, topoisomerases, ecdysone receptor, DNA polymerase subunits, RNA polymerase I, II and III subunits, cytoplasmic and nuclear factors.

The term "non-secretion competent heterologous proteins" means proteins which are not naturally secreted from the cell into the extracellular environment. Examples of non-secretion competent heterologous proteins are chloramphenicol acetyl transferase, human immunodeficiency virus (HIV-1) *gag, pol, sor, β*-galactosidase, *c-myc,* influenza polymerases PA, PB1, and PB2, *Neurospora crassa* activator protein, p53 protein, topoisomerases, ecdysone receptor, DNA polymerase subunits, RNA polymerase I, II and III subunits, cytoplasmic and nuclear factors and non-secretion competent subunits of secreted and non-secreted proteins.

The term "promoter" means a DNA sequence which initiates and directs the transcription of a heterologous gene into an RNA transcript in cells. The promoter may be a baculovirus promoter derived from any of over 500 baculoviruses generally infecting insects, such as for example the orders Lepidoptera, Diptera, Orthoptera, Coleoptera and Hymenoptera, including for example but not limited to the viral DNAs of *Autographa californica MNPV, Bombyx mori NPV, Tricoplusia ni MNPV, Rachiplusia ou MNPV, or Galleria mellonella MNPV* wherein said baculovirus promoter is a baculovirus immediate-early gene IE1 or IEN promoter; a delayed-early gene promoter region such as the 39K gene promoter or a baculovirus late gene promoter, such as the polyhedrin gene promoter. The promoter may also be a insect cellular promoter, such as the actin gene promoter, the ribosomal gene promoter, the histone gene promoter, or the tubulin gene promoter. The promoter may also be a mammalian promoter such as the cytomegalovirus immediate early promoter, the SV40 large T antigen promoter or the Rous Sarcoma virus (RSV) LTR promoter.

The term "enhancer" means a cis-acting nucleic acid sequence which enhances the transcription of the structural gent and functions in an orientation and position-independent manner. The enhancer can function in any location, either upstream or downstream relative to the promoter. The enhancer may be any DNA sequence which is capable of increasing the level of transcription from the promoter when the enhancer is functionally linked to the promoter, for example the RSV LTR enhancer, baculovirus HR1, HR2 or HR3 enhancers or the CMV immediate early gene product enhancer. In a preferred embodiment, the enhancer is the 1.2 kb BmNPV enhancer fragment set forth in U.S. Patent Application No. 08/608,617 which is incorporated by reference herein.

The term "signal sequence" or "leader sequence" means a polynucleotide which encodes an amino acid sequence, i.e. a "signal peptide" or "leader peptide", that initiates transport of a protein across the membrane of the endoplasmic reticulum. Signal sequences have been well characterized in the art and are known to typically contain 16 - 30 amino acid residues, but may contain greater or fewer amino acid residues. A consensus signal peptide consists of three regions: a basic N-terminal region, a central hydrophobic region, and a more polar C-terminal region. The central hydrophobic region contains 4 to 12 hydrophobic residues that anchor the signal peptide across the membrane lipid bilayer during transport of the nascent polypeptide. The signal peptide is usually cleaved within the lumen of the endoplasmic reticulum by cellular enzymes known as signal peptidases. Thus the portion of the DNA encoding the signal sequence may be cleaved from the amino terminus of the SCP-heterologous fusion protein during secretion. This results in production of the SCP-heterologous fusion protein consisting of the SC polypeptide fused to the heterologous protein. Suitable signal peptides or signal sequences include, but are not limited to, the JHE signal peptide, the GMCSF signal peptide, tissue plasminogen activator signal peptide; *Bombyx mori* chorion protein signal peptide, and the honey bee mellitin signal peptide. It is also contemplated that where a complete JHE protein is used for the secretion competent polypeptide, the complete protein may include its signal peptide. Therefore, another signal peptide sequence may not be necessary to achieve expression and secretion of the heterologous protein.

It is also contemplated that the expression of the heterologous gene may be enhanced by the expression of other factors, for example the IE-1 protein of nuclear polyhedrosis viruses or the herpes simplex virus VP 16 transcriptional activator.

The term "functionally linked" or "functionally attached" when describing the relationship between two DNA regions simply means that they are functionally linked to each other and they are located on the same nucleic acid fragment. A promoter is functionally attached to a structural gene if it controls the transcription of the gene and it is located on the same nucleic acid fragment as the gene. An enhancer is functionally linked to a structural gene if it enhances the transcription of that gene and it is functionally located on the same nucleic acid fragment as the gene.

The term "linked in frame" means that one gene is linked at its 3' end to the 5' end of a second gene such that after transcription and translation of the genes a single fusion protein comprising the two proteins encoded by the genes is produced. The two genes may be linked by a spacer nucleic acid sequence encoding amino acids.

The term "introduction" refers to either infection or transfection of insect cells.

The term "infection" refers to the invasion by pathogenic viral agents of cells where conditions are favorable for their replication. Such invasion can occur by placing the viral particles directly on the insect cell culture or by injection of the insect larvae with the recombinant virus or by oral ingestion of the viral particles by the insect. The amount of recombinant virus injected into the larvae will be from 10² to 10⁵ pfu of non-occluded virus/larvae. Alternatively, larvae can be infected by the oral route using occlusion bodies carrying recombinant viruses. In general, the amount of occlusion bodies fed to the larvae is that amount which for wild-type viruses corresponds to the LD₅₀ for that species of baculovirus and insect host. The LD₅₀ varies with each species of baculovirus and the age of the larvae. One skilled in the art can readily determine the amount of occlusion bodies to be administered. Typically, the amount will vary from 10-10⁶ occlusion bodies/insect.

The term "transfection" refers to a technique for introducing purified nucleic acid into cells by any number of methods known to those skilled in the art. These include but are not limited to, electroporation, calcium phosphate precipitation, lipofection, DEAE dextran, liposomes, receptor-mediated endocytosis, and particle delivery. The polynucleotide can also be used to microinject eggs, embryos or *ex vivo* or *in vitro* cells. Cells can be transfected with the polynucleotide described herein using an appropriate introduction technique known to those in the art, for example, liposomes. In a preferred embodiment, the polynucleotide is introduced into the insect cells by mixing the DNA solution with Lipofectin™ (GIBCO BRL Canada, Burlington, Ontario) and adding the mixture to the cells.

The term "insect cells" means insect cells from the insect species which are subject to baculovirus infection. For example, without limitation: *Autographa californica; Bombyx mori; Spodoptera frugiperda; Choristoneura fumiferana; Heliothis virescens; Heliothis zea; Helicoverpa zea; Helicoverpa virescens; Orgyia pseudotsugata; Lymantria dispar; Plutella xylostella; Malacostoma disstria; Trichoplusia ni; Pieris rapae; Mamestra configurata; Mamestra brassica; Hyalophora cecropia.*

### Methodology

Signal peptide sequences are often not sufficient for the efficient secretion of certain peptides or proteins such a nuclear factors from eukaryotic cells. Such peptides or proteins are termed non-secretion competent proteins.

It has now been found that the fusion of secretion competent polypeptide comprising the complete amino acid sequence of insect JHE or a portion thereof as defined above to the 5' end of a non-secretion competent protein will allow efficient secretion of the fusion protein from the cell into the extracellular environment. In order to achieve continuous high level secretion of heterologous proteins in cells, the cells are transformed with an expression cassette comprising a promoter functionally linked to a signal sequence which in turn is linked in frame to sequence encoding the secretion competent polypeptide which in turn is linked in frame to the gene coding for the heterologous protein. The linkage of the secretion competent polypeptide gene to the heterologous gene is preferably in frame to ensure that both the SCP gene and the heterologous gene are transcribed and translated as a single fusion protein.

To achieve continuous secretion of heterologous proteins, in one embodiment, normal insect tissue culture cells can be transformed with a vector containing such an expression cassette comprising a promoter, a signal sequence functionally linked in frame to the SCP gene as defined above which in turn is functionally linked in frame to the desired heterologous gene. It is contemplated that the vector may also contain an extra gene expressing a selective marker (e.g. antibiotic resistance gene under the control of a promoter that functions constitutively in insect cells). Application of a relevant selection should lead to integration of one or more multiple copies of the plasmid into the chromosomes of the insect cells, thus generating an insect cell line capable of continuous secretion of the heterologous protein.

It is contemplated that the expression cassette may also include an enhancer sequence which would increase the level of transcription from the promoter. The level of transcription from the cellular promoter functionally linked to an enhancer as compared to the level of transcription from the cellular promoter alone is preferably at least about 10 fold and more preferably at least about 100 fold.

The insect cells may further express other transcription factors which enhance transcription such as the IE-1 protein. In one embodiment the insect cells can be transformed with a vector containing the IE-1 gene and a suitable resistance/selectable marker gene. Application of a relevant selection should lead to integration of one or more multiple copies of the vector into the chromosomes of the cells, thus generating an insect cell line capable of continuous high level expression of the IE-1 gene product. Thus the cell line will contain the IE-1 gene in the absence of added baculovirus. Such a cell line can be subsequently transformed with additional vectors containing either the expression-cassette containing an insect cellular promoter functionally linked to the JHE gene and a heterologous gene. The second vector may also comprise an additional gene conferring resistance to a second selection agent. In another embodiment, the gene for the IE-1 protein may be inserted into the vector comprising the expression cassette such that the vector contains both the JHE-heterologous genes and the IE-1 gene. In both cases, synthesis of the foreign protein will be continuous, because integrated expression cassettes cannot be lost through replication and the insect cells never die because they are not infected by any viruses. The level of production of heterologous proteins in cells expressing the IE-1 gene as compared to cells without the IE-1 gene is preferably at least about 10 fold greater and more preferably at least about 100 fold greater.

The vector may be a baculovirus artificial chromosome as set forth in U.S. Patent Application Serial No. 60/056,807, entitled BACULOVIRUS ARTIFICIAL CHROMOSOMES AND METHODS OF USE, Attorney Docket No. 028722-153, filed August 21, 1997 and incorporated by reference in its entirety. Such baculovirus artificial chromosomes would not integrate into the cellular chromosomes but rather replicate autonomously without killing the cells.

It is appreciated that the expression cassette of the present invention could be used to express and secrete any heterologous protein. However, the expression cassette is particularly useful in the expression and secretion of heterologous proteins previously thought to not be secretion competent.

Mammalian cells could be transfected with an expression cassette of the present invention where the SCP is the juvenile hormone esterase secretion competent peptide. Methods of transfecting mammalian cells are known in the art. If mammalian cells were used, the promoter and enhancer sequences would preferably be those promoters and enhancer sequences suitable for expression of a heterologous gene in the mammalian cell. The heterologous protein would be secreted from the mammalian cell into the extracellular environment as a fusion protein, wherein the heterologous protein is fused in frame to the carboxyl terminus of the JHE peptide.

The heterologous protein is secreted from the insect cell into the extracellular environment as a fusion protein, wherein the heterologous protein may be fused in frame directly, or via a linking peptide, to the carboxyl terminus of the SC polypeptide. The heterologous fusion protein may then be treated to remove the SC polypeptide resulting in an active heterologous protein. In order to facilitate the removal of the SC polypeptide, it is contemplated that the heterologous gene may be linked to the SCP gene in frame via a linking sequence which encodes an amino acid sequence or linking peptide which can be easily cleaved. An example of a suitable cleavage site is the nucleic acid sequence coding for the amino acid sequence DDDDK, which is a cleavage site recognized by the protease porcine intestine enteropeptidase.

The linking sequence may also contain a DNA sequence encoding a spacer peptide for better access to the cleavage site. The linking sequence may also contain a sequence for efficient purification of the fusion peptide from the extracellular environment. An example of such a sequence is a nucleic acid sequence coding for six histidine residues, which residues will bind to a Ni(II)-NTA chromatography matrix for affinity purification.

### Utility

This technique would be useful for the extracellular production of non-secretion competent polypeptides from an insect cell for medical, research or veterinary application.

As can be appreciated from the disclosure above, the present invention has a wide variety of applications. Accordingly, the following examples are offered by way of illustration and not by way of limitation.

### EXAMPLES

In the examples below, the following abbreviations have the following meanings. If not defined below, then the abbreviations have their art recognized meanings.
- ORF -: open reading frame
- kb -: kilobase
- mg -: milligram
- mL -: milliliter

Chemicals used in the following examples were obtained from the following companies:
Amersham Canada Ltd., Oakville, Ontario, Canada
J.T. Baker, Phillipsburg, New Jersey
BioRad Laboratories Ltd. Canada, Mississauga, Ontario, Canada
Boehringer Mannheim, Laval, Quebec, Canada
5 Prime-3 Prime, Inc., Boulder, Colorado
GIBCO BRL Canada, Burlington, Ontario, Canada
Hyclone Laboratories, Inc., Logan, Utah
ICN Biopharmaceuticals Canada Ltd., Montreal Quebec, Canada
JRH Biosciences, Inc., Lenexa, Kansas
Life Technologies, Burlington, Ontario, Canada
New England Biolabs, Inc., Mississauga, Ontario, Canada
Pharmacia LKB, Baie d' Urfe' , Quebec, Canada
Promega Corporation, Madison, Wisconsin
Sigma, St. Louis, Missouri
Stratagene, La Jolla, California
United States Biochemicals, Cleveland, Ohio

All enzymes used for the construction and characterization of the recombinant plasmids and baculoviruses were obtained from Pharmacia, LKB; New England Biolabs, Inc.; GIBCO BRL Canada; Boehringer Mannheim; and used according to those suppliers recommendations.

The cloning procedures set forth in the examples are standard methods described in Sambrook et al., *Molecular Cloning: A Laboratory Manual,* Cold Spring Harbor Laboratory (1989) which is incorporated herein by reference. This reference includes procedures for the following standard methods: cloning procedures with *E. coli* plasmids, transformation of *E. coli* cells; plasmid DNA purification, agarose gel electrophoresis, restriction endonuclease digestion, ligation of DNA fragments and other DNA-modifying enzyme reactions.

### Example 1. Secretion of chloramphenicol acetyl transferase (CAT)

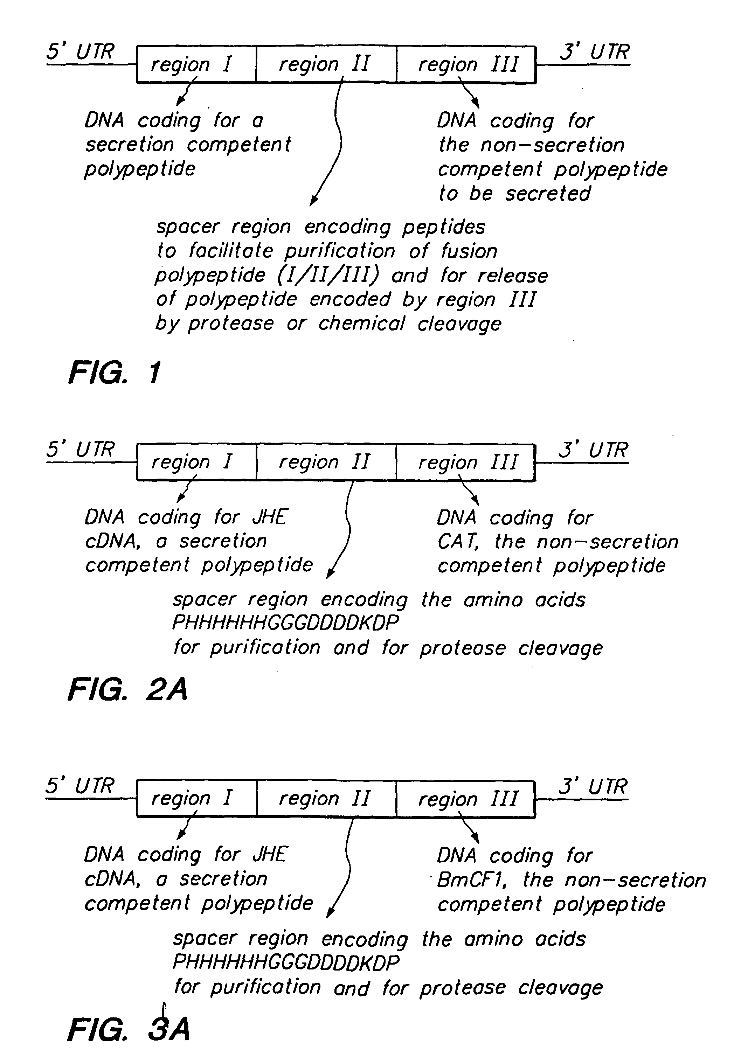

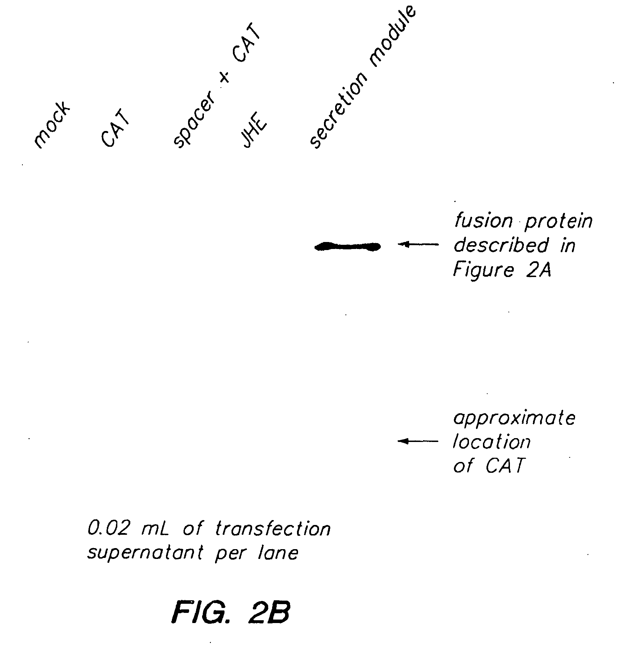

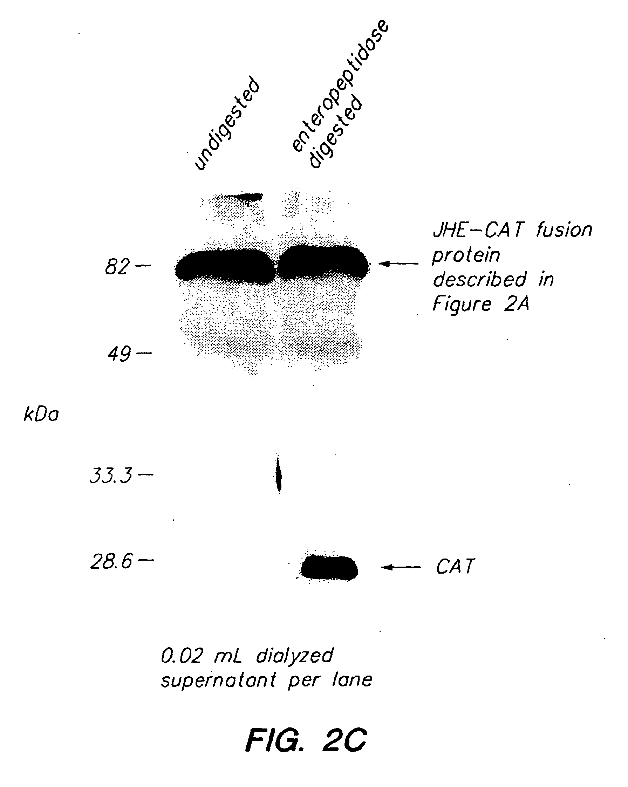

The DNA module for the secretion of chloramphenicol acetyl transferase (CAT) is shown in Figure 2A. At the 5' end, it contains the complete cDNA coding for the insect secreted protein juvenile hormone esterase (JHE) (Hanzlik et al.) which can be secreted from animal cell hosts. The spacer region contains DNA coding for six histidine residues that are attracted to Ni(II)-NTA chromatography matrices for affinity purification (Kroll 1993). The spacer region also contains a nucleic acid sequence coding for the amino acid sequence DDDDK, which is a cleavage site recognized by the protease porcine intestine enteropeptidase (Kell 1971). The spacer region is bound on each side by a proline residue which encourages the spacer peptide to form its own domain for better access to both the chromatographic purification matrix and the enteropeptidase. The module also contains the DNA sequence coding for CAT.

The vectors for Example 1 were constructed as follows. The expression plasmid pIE1/153A contains the *Bombyx mori* cytoplasmic actin cassette (Johnson et al., 1992; U.S. Patent Application No. 08/608,617), *Bombyx mori* Nuclear Polyhedrosis Virus (BmNPV) HR3 enhancer element and the BmNPV *ie1* gene and was constructed as follows. A 1.2 kb SspI fragment corresponding to the BmNPV genomic region from 51.8 to 52.7 map units containing the BmNPV HR3 element was cloned into the SmaI site of pBluescript-SK+ (Stratagene) to yield plasmid p153. The plasmid pIE1/153 was made by inserting a 3.8 kb ClaI fragment containing the *ie1* gene into the ClaI site of plasmid p153, removing unwanted restriction sites in the polylinker of this plasmid by double digestion with SacII and BamHI, blunt ending with T4 DNA polymerase and self-ligating the resultant plasmid. A 2.2 kb SacI fragment containing the actin cassette from the plasmid pBmA (Johnson et al., 1992) was ligated into the unique SacI site of plasmid pIE1/153 to yield the expression plasmid pIE1/153A.

The vector, pBmA is a pBluescript (Stratagene) derivative of clone pA3-5500 which contains the A3 cytoplasmic actin gene of *Bombyx mori* (Mounier and Prudhomme, 1986). Plasmid pBmA was constructed to contain 1.5 kb of the A3 gene 5' flanking sequences and part of its first exon to position +67 (relative to transcription initiation), a polylinker region derived from plasmid pBluescript (Stratagene) for insertion of foreign gene sequences, and an additional 1.05 kb of the A3 gene sequences encompassing part of the third exon of the gene from position + 836 and adjacent 3' flanking sequences which contain signals required for RNA transcript polyadenylation. See U.S. Patent Application Serial No.08/608,617 which is incorporated by reference herein in its entirety.

This expression vector was constructed by (1) subcloning into plasmid Bluescript-SK+ (Stratagene) a 1.5 kb Kpnl/AccI fragment of clone pA3-5500 containing the 5' flanking, 5' untranslated and coding sequences of the A3 gene up to position + 67 to generate plasmid pBmAp; (2) mutagenizing the ATG translation initiation codon present at position +36 to +38 of the actin coding sequence in plasmid pBmAp into AGG, AAG or ACG by the method of Kunkel (1985) to generate plasmids pBmAp.AGG, pBmAp.AAG and pBmAp.ACG; (3) subcloning into plasmid pSP72 (Promega Corporation) a 1.05 kb XhoI/SalI fragment of clone pA3-5500, containing part of the third exon of the actin gene from position +836 and adjacent 3' flanking sequences which include signals required for RNA transcript polyadenylation, to generate plasmid pBmAt; (4) converting the unique XhoI site of plasmid pBmAt into a NotI site by digestion of this plasmid with XhoI (GIBCO BRL), and end-filling with Klenow DNA polymerase (Boehringer Mannheim), ligation of NotI linkers (DNA Synthesis Laboratory.

A 0.8 kb BamHI fragment, containing the CAT open reading frame was isolated from *pBmA.CAT* (Johnson et al., 1992; U.S. Patent No. 08/608,617) and cloned into the unique BamHI site in *pIE1*/*153A* to generate *pIE1*/*153A. CAT.*

A 1.8 kb EcoRI fragment containing the JHE(kk) open reading frame was first isolated from *pAcUW21-KK* (Bonning and Hammock, 1996), NotI linkers were ligated to its ends, and it was inserted into the unique NotI site of *pIE1*/*153A* to generate the plasmid *pIE1*/*153A.JHE.*

The plasmid *pIE1*/*153A. JHE. HisEP. CAT* was generated in several steps.
(i) First 2 oligonucleotides [SEQ ID Nos: 1 and 2] were synthesized (5' to 3') coding for region II in Figure 2A:
   I.
   II.

   These oligonucleotides were annealed together, end-filled by mutually primed synthesis with Klenow enzyme, double digested with BamHI and NcoI, and ligated into *pBluescript-SK+* (Stratagene) to yield *pHisEP(NcoI).*
(ii) Next two mutagenic primers [SEQ ID Nos: 3 and 4] (5' to 3') were synthesized in order to generate region III in Figure 2A:
   I.
   II.

   PCR amplification using *Pfu* polymerase from *pIE1*/*153A. CAT* plasmid DNA yielded a 0.8 kb product containing the CAT open reading frame that was double digested with NcoI and XbaI and ligated in-frame into the unique NcoI/Xbal sites of *pHisEP(Ncol)* to yield *pHisEP. CAT.*
(iii) The following two mutagenic primers [SEQ ID Nos: 5 and 6] (5' to 3') were synthesized to obtain region I in Figure 2a:
   I.
   II.

   PCR amplification using *Pfu* polymerase from *pIE1*/*153A.JHE(kk)* plasmid DNA yielded a 1.6 kb product containing the JHE open reading frame (with no stop codon) that was partially digested with BamHI and ligated in-frame into the unique BamHI site of *pHisEP. CAT* to yield *pJHE.HisEP. CAT.*
(iv) A partial BamHI digestion and complete NotI digestion of *pJHE.HisEP.CAT* released a 2.5 kb fragment containing the complete secretion module (regions I, II, and III in Figure 2A) that was ligated into the unique BamHI/NotI sites of the expression plasmid *pIE1*/*153A* to yield *pIE1*/*153A. JHE.HisEP. CAT.*

Control DNA for the experimental demonstration of the secretion of CAT was the expression plasmid *pIE*/*153A* ("mock DNA "); the expression plasmid with the complete CAT gene *pIE1*/*153A. CAT (*"CAT"), the expression plasmid with spacer plus the CAT gene ("spacer + CAT") and the expression plasmid with the JHE gene *pIE1*/*153A.JHE* ("JHE") in Figure 2B.

The various expression plasmids were transfected into *Bonibyx mori* insect host cells in *in vitro* cultures. (Lu et al. 1996) Bm5 cells were maintained in IPL-41 (Gibco) + 10 % fetal bovine serum. For transfection, cells were seeded into 35mm diameter dishes at a density of 10⁶ cells/well, allowed to adhere, and transfected with 0.5 mL of basal media containing 3 micrograms plasmid DNA and 15 microgram Lipofectin (Gibco) for 5 hours according to manufacturers instructions. Cells and supernatant were harvested for analysis 2 days following transfection.

The extracellularly expressed CAT was detected by western blotting (Sambrook 1989) the culture supernatants, using an antibody recognizing CAT. Aliquots of cells or cell culture supernatants were resolved by electrophoresis in a SDS-containing 8% polyacrylamide gel (SDS-PAGE) and electroblotted onto Hybond-ECL membrane (Amersham). After transfer, the membrane was blocked for 1 hour at room temperature in 50 ml PBS-0.1 % Tween-20 (PBST) containing 10 % (w/v) skim milk powder (PBSTM). The filter was incubated for 1 hour at room temperature in 5 mL PBSTM containing rabbit polyclonal anti-CAT antibody (5 Prime-3 Prime, Inc., 1:1000 dilution), washed twice for 15 minutes with PBST, and incubated 1 hour with 5mL PBSTM containing horseradish peroxidase conjugated goat anti-rabbit IgG (Life Technologies; 1:1000 dilution). After washing twice with PBST, the filter was incubated with ECL chemiluminescent substrate (Amersham) according to the suppliers' instructions and exposed to X-ray film.

Figure 2B shows that no CAT was detected in either the supernatant of cells transfected with mock plasmid DNA or cells transfected with a plasmid expressing CAT or cells transfected with a plasmid expressing the spacer plus the CAT gene, or cells transfected with a plasmid expressing JHE. CAT was detected in the supernatant of cells transfected with plasmid *pIE1*/*153A.JHE.HisEP.CAT* ("secretion module"). Therefore, the naturally secreted protein JHE can be employed to drag a non-secretion competent polypeptide, such as CAT, into an extracellular environment.

### Example 2. Liberation of the CAT: peptide from the fusion protein

To demonstrate that the CAT protein could be liberated from the expressed fusion protein culture, supernatant from the culture described in Example 1 was dialyzed against enteropeptidase buffer, and incubated with porcine intestine enteropeptidase [ICN Biopharmaceuticals Canada Ltd.] for 36 hours at 37°C (Kell 1971). Figure 2C is a western blot of an enteropeptidase digested sample, using the anti-CAT antibody (5 Prime-3 Prime, Inc. 1:1000 dilution) which shows that some CAT was successfully liberated from the fusion protein.

### Example 3. Secretion of BmCF1

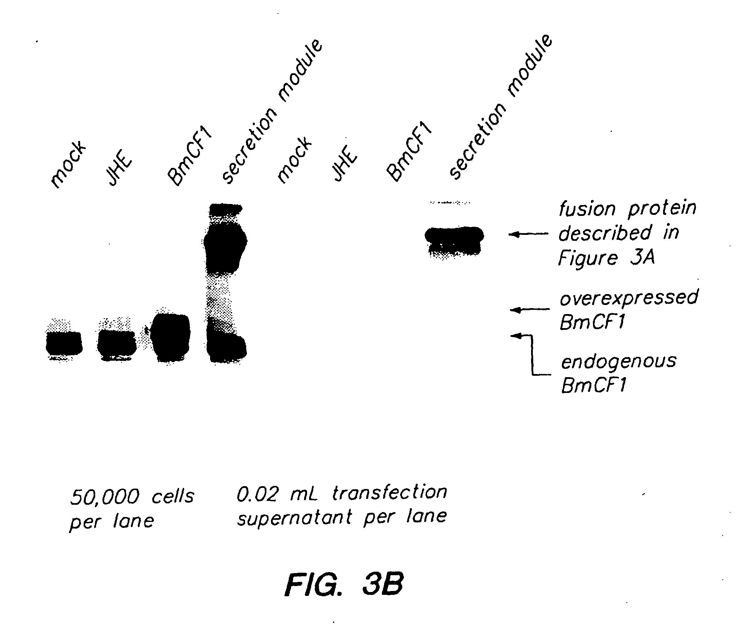

The intracellular protein *Bombyx mori* chorion factor 1 (BmCF1) is naturally found in the nucleus of some *Bombyx mori* insect cells. The module for secretion of BmCF1 is shown in Figure 3A.

A 3.8 kb NotI fragment of *pBmCFI* (Tzertziniz et al, 1994) containing the BmCF1 open reading frame was ligated into the unique NotI site of *pIE1*/*153A* to form *pIE1*/*153A.BmCF1.*

The plasmid *pIE1*/*153A.JHE.HisEP.BmCF1* was generated in several stages.
(i) First 2 oligonucleotides [SEQ ID Nos: 1 and 7] were synthesized (5' to 3') coding for region II in Figure 3A:
   I.
   II.

   These oligonucleotides were annealed together, endfilled by mutually primed synthesis with Klenow enzyme, double digested with BamHI and SphI and ligated into *pBtuescript-SK+* (Stratagene) to yield *pHisEP* (*SphI*).
(ii) The following 2 oligonucleotides [SEQ ID Nos: 8 and 9] were synthesized (5' to 3') to obtain region III in Figure 3A:
   I.
   II.

   PCR amplification using *Pfu* polymerase from *pBmCF1* plasmid DNA yielded a 1.5 kb product containing the BmCF1 open reading frame that was completely digested with XbaI and partially digested with SphI and ligated in-frame into the unique SphI/XbaI sites of *pHisEP (SphI)* to yield *pHisEP.BmCF1.*
(iii) The PCR product containing the JHE ORF (with no stop codon), described in Eample 1, was ligated in-frame into the unique BamHI site of *pHisEP.BmCFI* to yield *pJHE.HisEP.BmCF1.*
(iv) A partial BamHI digestion and complete NotI digestion of *pJHE.HisEP.BmCF1* released a 2.6 kb fragment containing the complete secretion module (regions I, II and III in Figure 3A) that was ligated into the unique BamHI/NotI sites of *pIEI1*/*153A* to yield *pIE1*/*153A.JHE. HisEP.BmCF1 .*

Control DNA for the experimental demonstration of the secretion of BmCF1 was the expression plasmid *pIE1*/*153A* ("mock DNA"); the expression plasmid with the complete BmCF1 gene, *pIE1*/*153A.BmCF1* ("BmCF1 "), and the expression plasmid with the complete JHE gene, *pIE1*/*153A.JHE* ("JHE") in Figure 3B.

Each plasmid was transfected into *Bombyx mori* insect host cells in *in vitro* cultures as set forth in Example 1. Intracellular and extracellular expressed BmCF1 was detected by western blotting using mouse monoclonal anti-BmCF1 (provided by Dr. F.C. Kafatos, Harvard University, Boston, Massachusetts; 1:100 dilution) and horse-radish peroxidase conjugate goat anti-mouse antibody [Life Technologies; 1:1000 dilution] by the methods set forth in Example 1.

The western blot, shown in Figure 3B reveals that the normally intracellular protein BmCF1 was only detected in the supernatant of cells transfected with the pIE1/153A.JHE.HisEP.BmCF1, ("secretion module") described in Figure 3A.

### Example 4 Secretion of JHE-CAT from mammalian cells

To demonstrate that the secretion module can be used to secrete a non-secretion competent polypeptide from mammalian cells a mammalian expression vector was employed. The bacterial cytoplasmic protein CAT was used an example of a non-secretion competent polypeptide.

Two vectors were constructed:
1) The vector pcDNA3.1.CAT was constructed by isolating the 800 bp BamHI fragment from pBmA.CAT containing the CAT open reading frome and cloning it into the unique BamHI site of the mammalian expression plasmid pcDNA3.1 + (Invitrogen, San Diego, CA).
2) The vector pcDNA3.1.JHE.HisEP.CAT was constructed as follows. A partial BamHI digestion and complete NotI digestion of pJHE.HisEP.CAT released a 2.5 kbp fragment containing the complete secretion module (regions I, II and III in Figure 2A) that was ligated into the unique BamHI/NotI sites of the mammalian expression plasmid pcDNA3.1 + (Invitrogen, San Diego CA) to yield pcDNA3.1.JHE.HisEP.CAT.

These vectors are used for the transfection of the mammalian cell line BHK-21, derived from baby hamster kidney cells in *in vitro* cell cultures. BHK-21 cells are maintained in DMEM (Gibco-BRL) plus 10% fetal bovine serum. The vectors are transfected into the BHK-21 cells by the method set forth in Example 1, except the cell density is 0.5 x 10⁶ cells/mL.

Following transfection with the plasmids pcDNA3.1 +, pcDNA3.1.CAT and pcDNA.JHE.HisEP.CAT a Western blot will reveal that the JHE-CAT fusion protein is secreted into the culture supernatant, while the CAT protein is not. This demonstrates that the naturally secreted JHE protein can be employed to secrete non-secretion competent polypeptides, such as CAT, into à extracellular environment.

### Example 5 Secretion of Bacterial Chloramphenicol Acetyl-transferase Using Human Granulocyte Macrophage Colony Stimulating Factor (Reference Example)

The human granulocyte macrophage colony stimulating factor (GMCSF) gene was also used for the secretion of CAT.

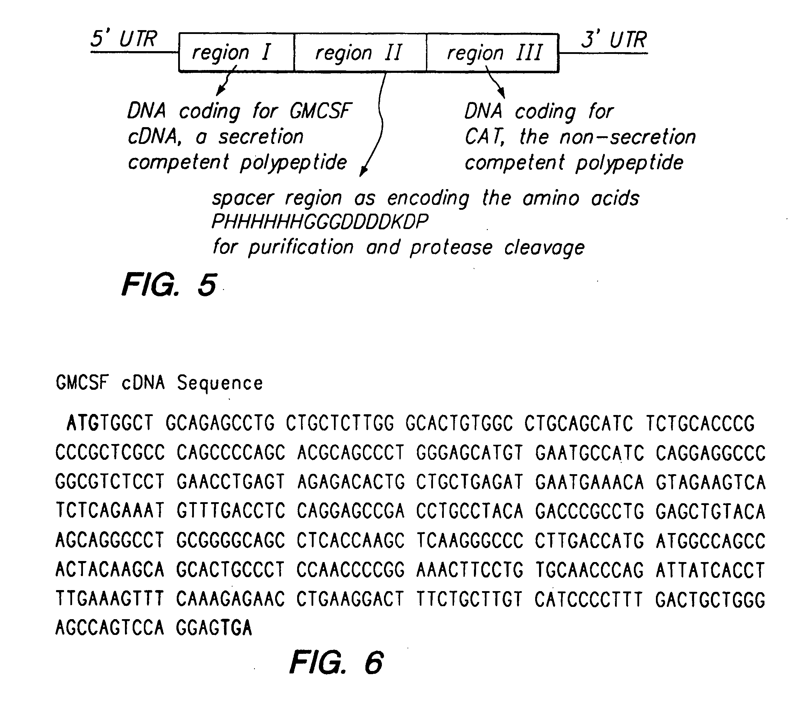

Figure 5 is a schematic illustration of the DNA encoding the fusion protein. At the 5' end it contains the complete cDNA coding for human granulocyte macrophage colony stimulating factor which can be secreted from animal cell hosts. It then contains a spacer region described in Example 1 and the DNA sequence coding for CAT linked in frame. The sequence of human GMCSF is shown in Figure 6. The normal start codon (ATG) and stop codon (TGA) are highlighted in bold.

The vector *pIE1*/*153A.GMCSF.HisEP.CAT* for Example 4 was constructed in several steps.
(i) First the vector *pIE1*/*153A.JHE.HisEP.CAT* was digested with BamHI to release the DNA coding for juvenile hormone esterase and yield an open vector *pIE1*/*153A.HisEP.CAT* (BamHI sticky ends).
(ii) Next two mutagenic primers [SEQ ID Nos: 10 and 11] (5' to 3') were synthesized:
   I.
   II.

PCR amplification using *Pfu* polymerase from *pGMCSF* (containing the complete GMCSF cDNA and provided by Dr Chris Brown, University of Calgary) plasmid DNA yielded a 450 bp product containing the complete human granulocyte macrophage colony stimulating factor (GMCSF) open reading frame (with no stop codon) that was digested with BamHI. This fragment was ligated into the open vector *pIE1*/*153A.HisEP.CAT* (BamHI sticky ends) to yield the vector *pIE1*/*153A.GMCSF.HisEP.CAT.*

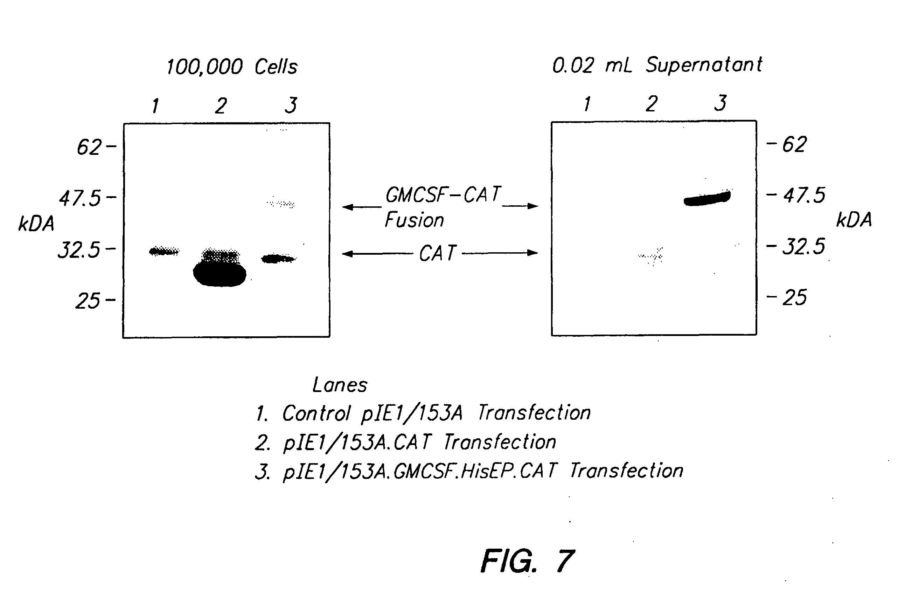

The expression plasmids *pIE1*/*153A* (control), *pIE1*/*153A.CAT* and *pIE1*/*153A. GMCSF.HisEP. CAT* were transfected into Bm5 *Bombyx mori* insect host cells in *in vitro* cultures as set forth in Example 1. Intracellular and extracellular CAT was detected by western blotting as set forth in Example 1. The western blot, shown in Figure 7 reveals that significantly more CAT present as a GMCSF-CAT fusion protein (over 100 fold as determined by densitometric scanning) was detected in the supernatant of cells transfected with the plasmid *pIE1*/*1153A.GMCSF.HisEP.CAT* than the supernatant of cells transfected with the plasmid *pIE1*/*153A.HisEP.CAT.*

While the present invention has been described with reference to what are considered to be the preferred examples, it is to be understood that the invention is not limited to the disclosed examples.

### REFERENCES

U.S. Patent Application No. 08/608,617
Bonning and Hammock, (1996) *Ann. Rev. Entomol.* **41**:191-210
Brinster et al., (1988) *Proc. Natl. Acad. Sci.* U.S.A. **85**:836-840
Davis and Wood (1995) "Intrinsic glycosylation potentials of insect cell cultures and insect larvae" in vitro Cell. Dev. Biol.
Garnier et al., (1994) *Cryotech* **15**(1-3):145-155
Hanzlik et al. , (1989) *J. Biol. Chem.***264***:12419-12425*
Huybrechts et al. (1992) "Nucleotide sequence of a transactivating *Bombyx mori* nuclear polyhedrosis virus immediate early gene" *Biochim. Bioph. Acta.* **1129**:328-330
Jarvis and Summers, (1989) "Glycosylation and secretion of human tissue plasminogen activator in recombinant baculovirus-infected cells." Mol Cell Biology 9:214-223
Johnson et al., (1992) "A cellular promoter-based expression cassette for generating recombinant baculoviruses directing rapid expression of passenger genes in infected insects" *Virology* **190**:815-823
Kell (1971) in *The Enzymes,* Academic Press, **3**:249-275
Kroll et al., (1993) *DNA and Cell Bio.* **12**:441-453
Kunkel (1985) *Proc. Nat. Acad. Sci* U.S.A. **82**:488-492
Lu et al., (1996)"trans-activation of a cell housekeeping gene promoter by the IE1 gene product of baculoviruses" *Virology* **218**:103-113
Maiorella et al. (1988) "Large scale insect culture media for recombinant protein production" *Bio*/*Technology* 6:1406-1410
Martens et al., (1995) "Characterization of baculovirus insecticides expressing tailored Bacillus thuringiensis CrylA(b) crystal proteins" *J. of Invertebrate Pathology* **66**:249-157
Mounier and Prudhomme, (1986) *Biochimie* **68**:1053-1061
O'Reilly et al., (1992) *Baculovirus Expression Vectors.* W.H. Freeman and Co.
Sambrook et al., (1989) In *Molecular Cloning, A laboratory Manual* Cold Spring Harbor Press.
Tzertziniz et al, (1994) *J. Mol. Biol.* **238**:479-486

## Claims

1. An expression cassette useful for the secretion of a heterologous protein as a fusion protein comprising a polynucleotide encoding from its 5' to 3' direction:
a) a promoter;
b) a signal peptide;
c) a cell secretion competent polypeptide which comprises the complete amino acid sequence of insect juvenile hormone esterase or a portion thereof, wherein said portion is capable of directing secretion of a non-secretion competent heterologous protein at a higher rate than the mere signal peptide of said juvenile hormone esterase; and
d) a heterologous protein
wherein the polynucleotide sequences encoding (b), (c) and (d) are linked in frame.

2. The expression cassette of claim 1 wherein the promoter sequence is selected from the group consisting of a viral promoter sequence, an insect cellular promoter sequence and a mammalian promoter sequence.

3. The expression cassette of claim 1 or 2 further comprising a polynucleotide sequence encoding an enhancer functionally linked to the promoter.

4. The expression cassette of claim 3, wherein the enhancer is a viral enhancer.

5. The expression cassette of any one of claims 1 to 4 wherein the sequence encoding the secretion competent polypeptide sequence is linked in frame to the sequence encoding the heterologous protein by a sequence encoding a linker peptide.

6. A vector useful for the secretion of a heterologous protein from eukaryotic cells comprising an expression cassette of any one of claims 1 to 5.

7. The vector of claim 5, further comprising a selectable marker gene.

8. A cell transformed with the expression cassette of any one of claims 1 to 5 or with the vector of claim 6 or 7.

9. The cell of claim 8 wherein the cell is from *Bombyx mori.*

10. An in vitro method of secreting a heterologous protein, comprising introducing into a cell an expression cassette comprising a polynucleotide encoding from its 5' to 3' direction:
a) a promoter;
b) a signal peptide;
c) a cell secretion competent polypeptide which is insect juvenile hormone esterase; and
d) a heterologous protein
wherein the polynucleotide sequences encoding (b), (c) and (d) are linked in frame under conditions wherein the heterologous protein is expressed and secreted from the cell.

11. The method of claim 10 wherein the promoter is selected from the group consisting of a viral promoter, an insect cellular promoter and a mammalian promoter.

12. The method of claim 10 or 11 wherein the expression cassette further comprises a DNA sequence encoding an enhancer functionally linked to the promoter.

13. The method of claim 12 wherein the enhancer is a viral enhancer.

14. The method of any one of claims 10 to 13 wherein the sequence encoding the secretion competent polypeptide sequence is linked in frame to the sequence encoding the heterologous protein by a sequence encoding a linker peptide.

15. The method of any one of claims 10 to 14 wherein said cell is a mammalian cell.

## Patentansprüche

1. Expressionskassette, die nützlich ist für die Sekretion eines heterologen Proteins als Fusionsprotein, umfassend ein Polynucleotid, das in seiner 5'- zu 3'-Richtung codiert:
(a) einen Promotor;
(b) ein Signalpeptid;
(c) ein zur Zellsekretion befähigtes Polypeptid, das die vollständige Aminosäuresequenz der juvenilen Hormon-Esterase von Insekten oder einen Teil davon umfaßt, wobei der Teil ein nicht zur Sekretion befähigtes heterologes Polypeptid mit einer höheren Rate als nur das Signalpeptid der juvenilen Hormon-Esterase zur Sekretion bringen kann; und
(d) ein heterologes Protein,
wobei die Polynucleotidsequenzen, die (b), (c) und (d) codieren, im Leserahmen verbunden sind.

2. Expressionskassette nach Anspruch 1, wobei die Promotorsequenz ausgewählt ist aus der Gruppe bestehend aus einer viralen Promotorsequenz, einer zellulären Promotorsequenz von Insekten und einer Promotorsequenz von Säugern.

3. Expressionskassette nach Anspruch 1 oder 2, die zusätzlich eine Polynucleotidsequenz umfasst, die einen Enhancer codiert, der funktionell mit dem Promotor verbunden ist.

4. Expressionskassette nach Anspruch 3, wobei der Enhancer ein viraler Enhancer ist.

5. Expressionskassette nach einem der Ansprüche 1 bis 4, wobei die Sequenz, welche die zur Sekretion befähigte Polypeptidsequenz codiert, im Leserahmen mit der Sequenz, die das heterologe Protein codiert, durch eine Sequenz, die ein Linkerpeptid codiert, verbunden ist.

6. Vektor, der für die Sekretion eines heterologen Proteins von eukaryontischen Zellen nützlich ist, umfassend eine Expressionskassette nach einem der Ansprüche 1 bis 5.

7. Vektor nach Anspruch 5, weiterhin umfassend ein selektierbares Markergen.

8. Zelle, transformiert mit der Expressionskassette nach einem der Ansprüche 1 bis 5 oder mit dem Vektor nach Anspruch 6 oder 7.

9. Zelle nach Anspruch 8, wobei die Zelle von *Bombyx mori* ist.

10. In-vitro-Verfahren zur Sekretion eines heterologen Proteins, umfassend das Einbringen einer Expressionskassette in eine Zelle, umfassend ein Polynucleotid, das in seiner 5'-zu 3'-Richtung codiert:
(a) einen Promotor;
(b) ein Signalpeptid;
(c) ein zur Zellsekretion befähigtes Polypeptid, das die juvenile Hormon-Esterase von Insekten ist; und
(d) ein heterologes Protein,
wobei die Polynucleotidsequenzen, die (b), (c) und (d) codieren, im Leserahmen verbunden sind, unter Bedingungen, bei denen das heterologe Protein exprimiert und von der Zelle sezerniert wird.

11. Verfahren nach Anspruch 10, wobei der Promotor ausgewählt ist aus der Gruppe bestehend aus einem viralen Promotor, einem zellulären Promotor von Insekten und einem Promotor von Säugern.

12. Verfahren nach Anspruch 10 oder 11, wobei die Expressionskassette zusätzlich eine DNA-Sequenz umfasst, die einen Enhancer codiert, der funktionell mit dem Promotor verbunden ist.

13. Verfahren nach Anspruch 12, wobei der Enhancer ein viraler Enhancer ist.

14. Verfahren nach einem der Ansprüche 10 bis 13, wobei die Sequenz, welche die zur Sekretion befähigte Polypeptidsequenz codiert, im Leserahmen mit der Sequenz, die das heterologe Protein codiert, durch eine Sequenz, die ein Linkerpeptid codiert, verbunden ist.

15. Verfahren nach einem der Ansprüche 10 bis 14, wobei die Zelle eine Säugerzelle ist.

## Revendications

1. Cassette d'expression utile pour la sécrétion d'une protéine hétérologue sous forme de protéine de fusion comprenant un polynucléotide codant dans le sens 5' vers 3' :
a) un promoteur ;
b) un peptide signal ;
c) un polypeptide capable de sécrétion cellulaire qui comprend la séquence d'acide aminé complète de l'estérase de l'hormone juvénile d'insecte ou une partie de celle-ci, ladite partie étant capable d'induire la sécrétion d'une protéine hétérologue incapable de sécrétion à un niveau plus élevé que le simple peptide signal de ladite estérase de l'hormone juvénile d'insecte ; et
d) une protéine hétérologue,
dans laquelle les séquences polynucléotidiques codant (b), (c), et (d) sont liées en phase.

2. Cassette d'expression selon la revendication 1, dans laquelle la séquence du promoteur est sélectionnée parmi le groupe consistant en une séquence de promoteur viral, une séquence d'un promoteur de cellule d'insecte et une séquence de promoteur mammifère.

3. Cassette d'expression selon la revendication 1 ou 2 comprenant en outre une séquence polynucléotidique codant un amplificateur liée de manière fonctionnelle au promoteur.

4. Cassette d'expression selon la revendication 3, dans laquelle l'amplificateur est un amplificateur viral.

5. Cassette d'expression selon l'une quelconque des revendications 1 à 4, dans laquelle la séquence codant la séquence du polypeptide capable de sécrétion est liée en phase à la séquence codant la protéine hétérologue par une séquence codant un peptide de liaison.

6. Vecteur utile pour la sécrétion d'une protéine hétérologue dans des cellules eucaryotes, comprenant une cassette d'expression selon l'une quelconque des revendications 1 à 5.

7. Vecteur selon la revendication 5, comprenant en outre un gène marqueur sélectionnable.

8. Cellule transformée avec une cassette d'expression selon l'une quelconque des revendications 1 à 5 ou avec un vecteur selon la revendication 6 ou 7.

9. Cellule selon la revendication 8, dans laquelle la cellule provient de *Bombyx mori.*

10. Méthode *in vitro* pour sécréter une protéine hétérologue, comprenant l'introduction dans une cellule d'une cassette d'expression comprenant un polynucléotide codant dans le sens 5' vers 3' :
a) un promoteur ;
b) un peptide signal ;
c) un polypeptide capable de sécrétion cellulaire qui est l'estérase de l'hormone juvénile d'insecte ; et
d) une protéine hétérologue ;
dans laquelle les séquences polynucléotidiques codant (b), (c), et (d) sont liées en phase, dans des conditions dans lesquelles la protéine hétérologue est exprimée et sécrétée par la cellule.

11. Méthode selon la revendication 10, dans laquelle le promoteur est sélectionné parmi le groupe consistant en un promoteur viral, un promoteur de cellule d'insecte et un promoteur mammifère.

12. Méthode selon la revendication 10 ou 11, dans laquelle la cassette d'expression comprend en outre une séquence d'ADN codant un amplificateur liée de manière fonctionnelle au promoteur.

13. Méthode selon la revendication 12, dans laquelle l'amplificateur est un amplificateur viral.

14. Méthode selon l'une quelconque des revendications 10 à 13, dans laquelle la séquence codant la séquence du polypeptide capable de sécrétion est liée en phase à la séquence codant la protéine hétérologue par une séquence codant un peptide de liaison.

15. Méthode selon l'une quelconque des revendications 10 à 14, dans laquelle ladite cellule est une cellule mammifère.


### SEQUENCE LISTING

(1) GENERAL INFORMATION
   (i) APPLICANT:
      (A) NAME: University Technologies International Inc.
      (B) STREET: 609 - 14^{th} Street
      (C) CITY: Calgary
      (D) STATE: Alberta
      (E) COUNTRY: Canada
      (F) POSTAL CODE. (ZIP): T2N 2A1
   (ii) TITLE OF INVENTION: SEQUENCES FOR IMPROVING THE EFFICIENCY OF SECRECTION OF NON-SECRETED PROTEINS FROM MAMMALIAN AND INSECT CELLS
   (iii) NUMBER OF SEQUENCES: 14
   (iv) CORRESPONDENCE ADDRESS:
      (A) NAME: Marks & Clerk
      (B) STREET: 55 Metcalfe Street, Suite 1380
      (C) CITY: Ottawa
      (D) STATE: Ontario
      (E) COUNTRY: Canada
      (F) POSTAL CODE (ZIP): KIP 6L5
      (G) TELEPHONE: 613-236-9561
   (v) COMPUTER READABLE FORM:
      (A) MEDIUM TYPE: Diskette, 3.5 inch, 1.44 MB
      (B) COMPUTER: IBM PC
      (C) OPERATING SYSTEM: Dos 5.0
      (D) SOFTWARE: Patent In Ver. 2.0
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER: 09/256,694
      (B) FILING DATE: 1999-02-24
      (C) CLASSIFICATION: Unknown
   (vii) PRIOR APPLICATION DATA:
      (A) APPLICATION NUMBER: 09/136,421
      (B) FILING DATE: 1998-08-20
      (C) CLASSIFICATION: Unknown
   (vii) PRIOR APPLICATION DATA:
      (A) APPLICATION NUMBER: 60/056,871
      (B) FILING DATE: 1997-0-21
      (C) CLASSIFICATION: Unknown
   (viii) PATENT AGENT INFORMATION:
      (A) NAME: Richard J. Mitchell
      (B) REGISTRATION NUMBER:
      (C) REFERENCE/DOCKET NUMBER: 98552-PCT
   (iv) TELECOMMUNICATION INFORMATION:
      (A) TELEPHONE: 613-236-9561
      (B) TELFAX: 613-230-8821
(2) INFORMATION FOR SEQ ID NO:1:
   (i) SEQUENCE CHARACTERISTICS:
      (A) LENGTH: 43
      (B) TYPE: DNA
      (C) TOPOLOGY: Artificial Sequence
   (ix) FEATURE:
      (A) OTHER INFORMATION: Encodes a portion of SEQ ID No.: 12
   (xi) SEQUENCE DESCRIPTION: SEQ ID NO:1:
   aaaggatcca atgccacatc atcatcatca tcatggcggc ggc                 43
(2) INFORMATION FOR SEQ ID NO:2:
   (i) SEQUENCE CHARACTERISTICS:
      (A) LENGTH: 42
      (B) TYPE: DNA
      (C) TOPOLOGY: Artificial Sequence
   (ix) FEATURE:
      (A) OTHER INFORMATION: Encodes a portion of SEQ ID No.: 12
   (xi) SEQUENCE DESCRIPTION: SEQ ID NO:2:
   aaaaccatgg cctgggtcct tgtcgtcgtc gtcgccgccg cc 42
(2) INFORMATION FOR SEQ ID NO:3:
   (i) SEQUENCE CHARACTERISTICS:
      (A) LENGTH: 28
      (B) TYPE: DNA
      (C) TOPOLOGY: Artificial Sequence
   (ix) FEATURE:
      (A) OTHER INFORMATION: Primer for PCR amplification
   (xi) SEQUENCE DESCRIPTION: SEQ ID NO:3:
   gggctaccat ggagaaaaaa atcactgg 28
(2) INFORMATION FOR SEQ ID NO:4:
   (i) SEQUENCE CHARACTERISTICS:
      (A) LENGTH: 29
      (B) TYPE: DNA
      (C) TOPOLOGY: Artificial Sequence
   (ix) FEATURE:
      (A) OTHER INFORMATION: Primer for PCR amplification
   (xi) SEQUENCE DESCRIPTION: SEQ ID NO:4:
   gggtgctcta gaatttctgc cattcatcc 29
(2) INFORMATION FOR SEQ ID NO: 5:
   (i) SEQUENCE CHARACTERISTICS:
      (A) LENGTH: 30
      (B) TYPE: DNA
      (C) TOPOLOGY: Artificial Sequence
   (ix) FEATURE:
      (A) OTHER INFORMATION: Primer for PCR amplification
   (xi) SEQUENCE DESCRIPTION: SEQ ID NO:5:
   aaaaggatcc atgacttcac acgtactcgc                30
(2) INFORMATION FOR SEQ ID NO:6:
   (i) SEQUENCE CHARACTERISTICS:
      (A) LENGTH: 29
      (B) TYPE: DNA
      (C) TOPOLOGY: Artificial Sequence
   (ix) FEATURE:
      (A) OTHER INFORMATION: Primer for PCR amplification
   (xi) SEQUENCE DESCRIPTION: SEQ ID NO:6:
   aaaaggatcc ttcaagcggg cttctactg                 29
(2) INFORMATION FOR SEQ ID NO:7:
   (i) SEQUENCE CHARACTERISTICS:
      (A) LENGTH: 42
      (B) TYPE: DNA
      (C) TOPOLOGY: Artificial Sequence
   (ix) FEATURE:
      (A) OTHER INFORMATION: Encodes a portion for SEQ ID NO.: 12
   (xi) SEQUENCE DESCRIPTION: SEQ ID NO:7:
   aaaagcatgc cctgggtcct tgtcgtcgtc gtcgccgccg cc  42
(2) INFORMATION FOR SEQ ID NO:8:
   (i) SEQUENCE CHARACTERISTICS:
      (A) LENGTH: 25
      (B) TYPE: DNA
      (C) TOPOLOGY: Artificial Sequence
   (ix) FEATURE:
      (A) OTHER INFORMATION: Primer for PCR amplification
   (xi) SEQUENCE DESCRIPTION: SEQ ID NO:8:
   tgtgggcatg cagagcgtgg cgaag 25
(2) INFORMATION FOR SEQ ID NO:9:
   (i) SEQUENCE CHARACTERISTICS:
      (A) LENGTH: 31
      (B) TYPE: DNA
      (C) TOPOLOGY: Artificial Sequence
   (ix) FEATURE:
      (A) OTHER INFORMATION: Primer for PCR amplification
   (xi) SEQUENCE DESCRIPTION: SEQ ID NO:9:
   cgacattcaa atctagaata agtcccccta c 31
(2) INFORMATION FOR SEQ ID NO:10:
   (i) SEQUENCE CHARACTERISTICS:
      (A) LENGTH: 26
      (B) TYPE: DNA
      (C) TOPOLOGY: Artificial Sequence
   (ix) FEATURE:
      (A) OTHER INFORMATION: Primer for PCR amplification
   (xi) SEQUENCE DESCRIPTION: SEQ ID NO:10:
   gaaggatccg atgtggctgc agagcc 26
(2) INFORMATION FOR SEQ ID NO:11:
   (i) SEQUENCE CHARACTERISTICS:
      (A) LENGTH: 26
      (B) TYPE: DNA
      (C) TOPOLOGY: Artificial Sequence
   (ix) FEATURE:
      (A) OTHER INFORMATION: Primer for PCR amplification
   (xi) SEQUENCE DESCRIPTION: SEQ ID NO:11:
   caaggatccc tcctggactg gctccc 26
(2) INFORMATION FOR SEQ ID NO:12:
   (i) SEQUENCE CHARACTERISTICS:
      (A) LENGTH: 17
      (B) TYPE: PRT
      (C) TOPOLOGY: Artificial Sequence
   (ix) FEATURE:
      (A) OTHER INFORMATION: Has a cleavage site recognized by the protease porcine intestine enteropeptidase
   (xi) SEQUENCE DESCRIPTION: SEQ ID NO:12:
   Pro His His His His His His Gly Gly Gly Asp Asp Asp Asp Lys Asp
    1               5                  10                  15
   Pro
(2) INFORMATION FOR SEQ ID NO:13:
   (i) SEQUENCE CHARACTERISTICS:
      (A) LENGTH: 1691
      (B) TYPE: DNA
      (C) TOPOLOGY: Heliothis virescens
   (xi) SEQUENCE DESCRIPTION: SEQ ID NO:13:
   atgacttcac acgtactcgc gctcgccttc cttctacacg cgtgcacagc gctggcgtgg   60
   caggagacaa attcgcgcag cgtggtcgcc catctggact ccggcattat acgcggcgtg  120
   ccgcgctcag cggatggcat caagttcgcc agcttcctag gagtgcccta cgctaagcag  180
   cctgttggag aactcaggtt taaggagctc gagcctctag aaccttggga taatatcctg  240
   aacgcaacaa atgaaggacc catctgcttc caaacagatg tattatacgg gaggctcatg  300
   gcggcaagcg agatgagcga ggcttgcata tacgccaaca ttcatgttcc atggcaaagc  360
   cttccccgag tgagggggac cacaccttta cggcctatcc tggtgttcat acatggtgga  420
   ggatttgctt tcggctccgg ccacgaggac ctacacggac cagaatattt ggtcactaag  480
   aatgtcatcg tcatcacgtt taattacaga ttgaacgtct tcggtttcct gtccatgaac  540
   acaacaaaaa tccccgggaa tgccggtctc cgggatcagg taaccctgtt gcgctgggtg  600
   caaaggaacg ccaagaattt cggaggagac cccagcgaca tcaccatagc ggggcagagc  660
   gctggtgcat cagctgcgca tctactgact ctttctaaag ctactgaagg tcttttcaaa  720
   agagcgattc tgatgagcgg aacaggaatg agctacttct ttactacttc tccacttttc  780
   gcggcctaca tttcgaaaca gttgttgcaa atcctgggca atcaacgaga cggatccgaa  840
   gaaatacatc ggcagctcat cgacctaccc gcagagaaac tgaacgaggc taacgccgtc  900
   ctgattgaac aaattggcct gacaaccttc ctccctattg tggaatcccc actacctgga  960
   gtaacaacca ttattgacga tgatccagaa atcttaatag ccgaaggacg cggcaagaat 1020
   gttccacttt taataggatt taccagctca gaatgcgaga ctttccgcaa tcgactattg 1080
   aactttgatc tcgtcaaaaa gattcaggac aatcctacga tcataatacc gcctaaactg 1140
   ttatttatga ctccaccaga gctgttgatg gaattagcaa agactatcga gagaaagtac 1200
   tacaacggta caataagtat cgataacttc gtaaaatcat gttcagatgg cttctatgaa 1260
   taccctgcat tgaaactggc gcaaaaacgt gccgaaactg gtggagctcc actgtacttg 1320
   taccggttcg cgtacgaggg tcagaacagc atcatcaaga aggtaatggg gctgaaccac 1380
   gagggtgtcg gccacattga ggacttaacc tatgtgttta aggtcaactc tatgtccgaa 1440
   gctctgcacg catcgccttc tgagaatgat gtgaaaatga agaatctaat gacgggctat 1500
   ttcttaaatt ttataaagtg cagtcaaccg acatgcgaag acaataactc attggaggtg 1560
   tggccggcta acaacggcat gcaatacgag gacattgtgt ctcccaccat catcagatcc 1620
   aaggagttcg cctccagaca acaagacatt atcgagttct tcgacagctt caccagtaga 1680
   agcccgcttg a                                                     1691
(2) INFORMATION FOR SEQ ID NO:14:
   (i) SEQUENCE CHARACTERISTICS:
      (A) LENGTH: 435
      (B) TYPE: DNA
      (C) TOPOLOGY: Human
   (xi) SEQUENCE DESCRIPTION: SEQ ID NO:14:
   atgtggctgc agagcctgct gctcttgggc actgtggcct gcagcatctc tgcacccgcc  60
   cgctcgccca gccccagcac gcagccctgg gagcatgtga atgccatcca ggaggcccgg 120
   cgtctcctga acctgagtag agacactgct gctgagatga atgaaacagt agaagtcatc 180
   tcagaaatgt ttgacctcca ggagccgacc tgcctacaga cccgcctgga gctgtacaag 240
   cagggcctgc ggggcagcct caccaagctc aagggcccct tgaccatgat ggccagccac 300
   tacaagcagc actgccctcc aaccccggaa acttcctgtg caacccagat tatcaccttt 360
   gaaagtttca aagagaacct gaaggacttt ctgcttgtca tcccctttga ctgctgggag 420
   ccagtccagg agtga                                                 435